Europäisches Patentamt

European Patent Office

Office européen des brevets

(19)

(11) Publication number: **0 101 887**
A2

(12)

# EUROPEAN PATENT APPLICATION

(21) Application number: 83107119.6

(22) Date of filing: 21.07.83

(51) Int. Cl.³: **A 61 K 7/09**
**A 61 K 7/48, C 09 K 15/00**

(30) Priority: 30.07.82 US 401882

(43) Date of publication of application:
07.03.84 Bulletin 84/10

(84) Designated Contracting States:
AT BE DE FR GB IT

(71) Applicant: Helene Curtis Industries, Inc.
4401 West North Avenue
Chicago, Ill. 60639(US)

(72) Inventor: Hsiung, Du Yung
130 Illinois Street
Park Forest Illinois 60 466(US)

(74) Representative: Reitzner, Bruno, Dr.
Patentanwälte Dipl.-Ing. R. Splanemann Dr. B. Reitzner
Tal 13
D-8000 München 2(DE)

(54) Cosmetic compositions comprising an aqueous solution of an urea compound, hair treatment, skin moisturizer, hair waving system, retardation of alkali production, and hair and skin treatment methods.

(57) A cosmetic composition containing stabilized aqueous urea present at about 0.5 to about 12 molar and containing a sufficient amount of an ammonium salt of an unreactive acid to adjust the pH value of the urea solution at about 6 to about 8 is disclosed, as are methods of its use.

EP 0 101 887 A2

Croydon Printing Company Ltd

4401 West North Avenue
Chicago, Ill. 60 639
U S A

Cosmetic Compositions Comprising an Aqueous Solution
of an Urea Compound, Hair Treatment, Skin Moisturizer,
Hair Waving System, Retardation of Alkali Production and
Hair and Skin Treatment Methods

## Technical Field

The present invention relates to cosmetic compositions that contain urea or a substituted urea, and more particularly to an aqueous composition and method in which the urea or the substituted urea is relatively stable to hydrolysis upon aging of the composition.

## Background Art

Urea and lower alkyl-substituted ureas such as N-methylurea are well known in the cosmetic arts, and particularly in the hair waving and straightening arts, as useful agents in promoting the treatment being carried out. For example, U.S. Patent No. 2,717,228 teaches that urea or mono-alkyl substituted ureas can be used at about 0.5 to about 4 molar with thiol compounds that are present at 0.35 to about 0.8 molar in hair waving compositions having a pH value between about 8.6 and about 9.5. This patent teaches that use of the urea permits a reduction in the amount of thiol present while maintaining a given amount of waving. Similarly, U.S. Patent No. 4,243,659 teaches the use of N,N'-dimethylurea as a swelling agent in cleansing compositions that contain the bisulfite ion along with detergent.

The beneficial attributes of the use of urea in cosmetic compositions are tempered by the fact that urea and its lower alkyl derivatives when dissolved in the aqueous cosmetic composition, tend to hydrolyze on storage to produce an alkali, such as ammonia or a lower alkyl-substituted amine. This hydrolysis of urea is evidenced by the teachings of U.S. Patent No. 3,847,165 wherein it is taught that a "two-stage system" is required for the use of urea

-2-

because of the instability of urea in solution over long periods of time. Similarly, U.S. Patent No. 4,243,659 teaches that cleansing compositions containing bisulfite ion and N,N'-dimethylurea are prepared at a pH in the range of 4.0 to 6.9 "to substantially prevent alkaline hydrolysis of the urea derivative." Still further, U.S. Patent No. 4,177,260 teaches that urea is not stable in aqueous solutions that contain sulfite ions at pH 6 and saponifies to yield ammonium carbonate. The saponification or hydrolysis of urea is said to be avoided in that latter patent by the use of a specific cyclic urea; i.e., ethylene urea or imidazolidinone-(2).

The problems associated with the hydrolysis of urea and its lower alkyl-substituted derivatives are avoided to some extent by the use of separately packaged, dry urea that is mixed into the cosmetic composition just prior to use. An example of the use of dry urea that is dissolved prior to use in a cosmetic composition can be found in U.S. No. 3,847,165.

While the use of dry urea tends to overcome the problem of hydrolysis of the aqueous urea, or its derivative, upon storage, that use also has several disadvantages. First, dissolution of a dry component into a solution is time consuming, inconvenient and sometimes incomplete. In addition, dissolution of urea in water is endothermic, leading to a considerable drop in the temperature of the resulting solution, and the use of such cold solutions in a hair treating, cosmetic composition such as in hair waving tends to retard the rate at which waving occurs.

On the other hand, if the useful urea were already in solution and used as such, the problems with urea dissolution would not be encountered.

-3-

Similarly, if two or more solutions need only to be admixed, that admixture is relatively easily done and will not produce as drastic a change in temperature as when the same amount of solid urea is dissolved to form the same amount of solution.

It is thus seen that if an aqueous solution of urea could be made stable to hydrolysis on storage, such an aqueous solution would be a great benefit to the cosmetics industry.

Summary of the Invention

The present invention relates to a cosmetic composition of an aqueous solution of an urea compound such as urea itself or a lower alkyl-substituted urea or mixtures thereof that is present in the solution at about 0.5 to about 12 molar. Also in the solution is an amount of an ammonium salt of an unreactive acid in sufficient quantity to adjust the pH value of the urea solution to about 6 to about 8. The amount of the ammonium salt of an unreactive acid that is typically useful ranges from about 0.02 molar to about 1 molar.

The stabilized cosmetic composition of this invention can be used alone and with added ingredients as a put-on leave-on treatment for providing increased weight, bulk, static control and moisturization to hair and in skin moisturizers. The stabilized cosmetic composition is also particularly useful as a separately packaged component of a hair waving system, another component of which includes an aqueous solution of a hair disulfide bond-breaking agent. These two components are admixed prior to use and then applied to the hair as a waving lotion.

The present invention has several benefits and advantages. One advantage is that it provides aqeuous urea solutions that exhibit reduced amounts of

-4-

titratable alkali that results from urea hydrolysis on aging at elevated temperatures.

One of the benefits of the present invention is that the use of urea in cosmetics can be obtained without the disadvantage of the urea being supplied as a solid or as a more costly cyclic compound.

Still further benefits and advantages of the present invention will become apparent to those skilled in the art from the detailed description of the invention and in the claims which follow.

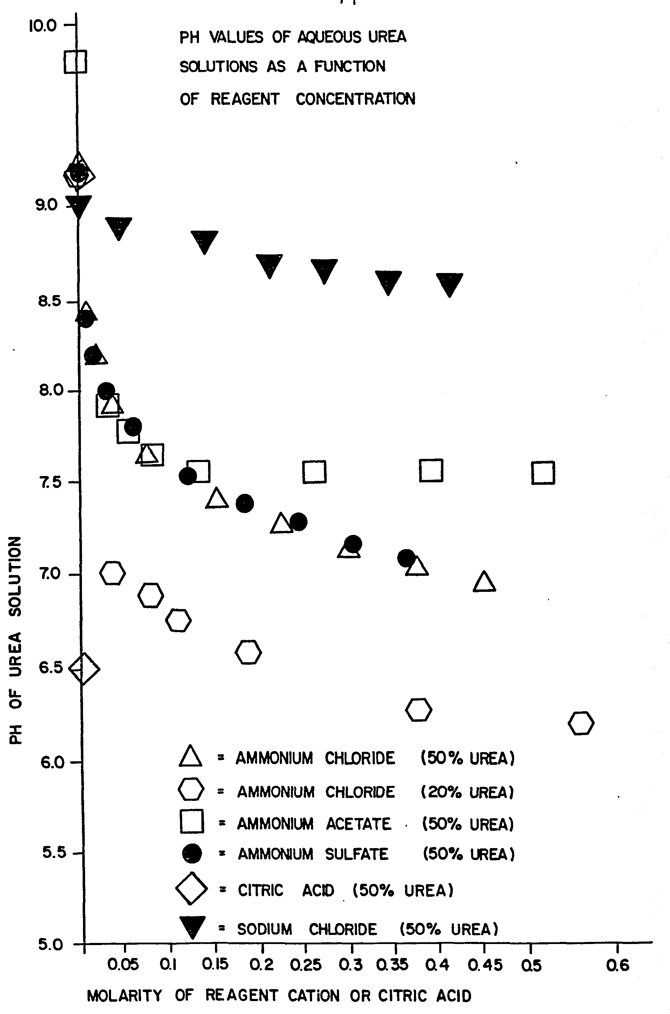

Brief Description of the Drawing

In the drawing, forming a portion of the disclosure of this invention;

The sole Figure is a graph showing the effect upon the pH value of aqueous urea solutions caused by the addition of differing amounts of reagents.

Detailed Description of the Invention

Urea and lower alkyl-substituted ureas are usually considered to be neither acids nor bases, but neutral compounds. However, because ureas are particularly susceptible to hydrolysis, dissolution of urea and lower alkyl-substituted ureas in water produces aqueous solutions having a basic pH value. For example, aqueous solutions that contain about 50 weight percent urea can have pH values as high as about 9 to about 10, probably because of hydrolysis of solid urea in the presence of moist air, as is shown in the graph of the Figure.

Dissolved in aqueous solution, urea and lower alkyl-substituted ureas hydrolyze substantially in a few weeks, and cannot therefore be used as such in commercial cosmetic products. It has now surprisingly been found that cosmetic compositions containing aqueous solutions of urea and lower alkyl-substituted ureas can be stabilized to hydrolysis for extended

-5-

periods of time, even when stored at elevated temperatures, by the presence of an ammonium salt of an acid that is chemically unreactive and present in an amount sufficient to adjust the pH value of the resulting solution to about 6 to about 8.

The phrases "stable", "stable to hydrolysis" and "stable to the production of titratable alkali" and the various grammatical forms of the words of those phrases are used herein to mean that the solution so characterized, which has a pH value of about 6 to about 8 and contains a urea compound and an ammonium salt of an unreactive acid, produces about one-half or less of the amount of titratable alkali than does a similar composition that contains the same amount of urea and is substantially free from an ammonium salt of an unreactive acid after both solutions are stored at 43° C. for a period of two months. A method for determining the amount of titratable alkali is shown in Example 1. The amount of titratable alkali produced is calculated by subtracting the amount of titratable alkali initially present from the amount found after the storage period.

The urea compounds useful in the cosmetic compositions of this invention include urea itself as well as the mono-, di-, tri-, and tetralower alkyl-N-substituted derivatives. The phrase "lower alkyl" is used herein to include alkyl groups that contain 1-4 carbon atoms. Thus, substituted ureas such as N-ethylurea, N-butyl-N'-propylurea, N,N'-dimethylurea, N,N,N'-trimethylurea, N,N,N',N'-tetraethylurea, and the like are contemplated, as are mixtures of the substituted ureas with themselves and with urea itself. Urea, itself, is most preferred herein and will be used illustratively hereinafter to represent the group of urea compounds.

-6-

Urea is preferably used in the cosmetic composition of this invention at a concentration of about 0.5 to about 12 molar, or at about 3 to about 65 weight percent of the cosmetic composition. In more preferred practice, the urea is present at about 6 to about 55 weight percent or about 1 to about 11 molar.

The cosmetic composition also includes a water-soluble ammonium salt of an unreactive acid. As used herein, the phrases "unreactive acid" and "acid that is chemically unreactive" denote acids and acid anions that do not oxidize or reduce urea or any other species present in the urea-containing solution or in a further composition with which the urea-containing solution is admixed. The "unreactive" acids contain no nucleophilic or electrophilic groups, nor are they themselves or their anions nucleophilic or electrophilic so as to catalyze or participate in the hydrolysis of urea. Still further, the "unreactive" acids or their anions do not form micellular or similar structures in water, nor are they surfactants.

Exemplary acid anions that are reactive from the oxidation-reduction viewpoint and are therefore excluded from the group of water-soluble unreactive acids include the chromate, permanganate, hypochlorate, nitrate, nitrite, peracetate, perborate, perbromate, phosphite, hypophosphite, sulfite, sulfide ions and the like. Exemplary acids and acid anions that are nucleophilic and are therefore excluded include thioglycolate, sulfite and bisulfite ions, amino acids, such as lysine and iso-leucine, hydroxamic acids such as acetohydroxamic acid and the like. Exemplary acid anions that form micellular or similar structures in water or are

-7-

surfactants and are excluded from being "unreactive" include those materials containing carbon chains of about 8 to about 20 carbon atoms and are terminated by a carboxylate, sulfonate, sulfate, phosphate or phosphonate group.

Water-soluble ammonium salts of unreactive acids that are useful herein include ammonium salts of strong acids; i.e., acids having $pK_a$ values of about 1 or less, and carboxylic acids that contain up to about four carbon atoms in the carbon chain.

Ammonium salts of strong acids are particularly preferred. Exemplary strong acids include hydrochloric, sulfuric, benzenesulfonic and pyrophosphoric acids. Most preferred ammonium salts of strong acids are ammonium chloride, ammonium sulfate, and mixtures thereof. Exemplary ammonium salts of carboxylic acids include ammonium acetate, ammonium propionate, diammonium succinate, diammonium methylsuccinate, ammonium bicarbonate and mixtures thereof.

The amount of the ammonium salt of an unreactive acid (hereinafter "ammonium salt") useful to provide the unexpected stability to the aqueous urea solution varies with the amount of urea in the cosmetic composition. One convenient way to measure the amount of ammonium salt needed is by the pH value of the resulting solution after the addition of the ammonium salt. Thus, it has been found that when an ammonium salt has been added to the aqueous urea solution in an amount sufficient to adjust the pH value of that solution to about 6 to about 8, the urea in the resulting solution is stabilized to the production of titratable alkali. More preferably, an amount of ammonium salt is added that is sufficient to adjust the pH value of the urea solution to about 6.5 to about 7.5.

-8-

Quantitatively, the amount of ammonium salt useful for adjusting the pH value of the cosmetic composition is about 0.02 to about 1 molar ($\underline{M}$). More preferably, the ammonium salt is present at about 0.1 to about 0.75 $\underline{M}$ in the cosmetic composition.

As can be seen from the graph of the Figure, there is not a straight line relationship between the molar concentration of ammonium ion and the change in pH value for the amount of urea shown. Nevertheless, it can be said that when more urea is present in solution, more ammonium salt is required to adjust the pH value to the desired range to obtain stabilization. The presence of less urea requires a smaller amount of ammonium salt to provide pH value adjustment and stability.

It is also noted from examination of the graph of the Figure that a salt of a strong acid and strong base such as sodium chloride does not change the pH value of a urea solution substantially. Small amounts of citric acid can be seen to cause drastic changes in pH value.

The mechanism by which the aqueous urea is stabilized by the ammonium salt is not understood. The improved stability is not believed to be due simply to the near neutral pH value of the urea-ammonium salt cosmetic compositions because, as is illustrated in Tables 1 and 2 of Example 1, solutions having the same amount of urea and that are within the pH value range of the cosmetic compositions herein are not stable.

It is believed that increased ionic strength or molality caused by the addition of the ammonium salt does not cause the observed stability of urea since a similar amount of sodium chloride does not lead to a relative decrease in the production of

-9-

titratable alkali.  This is shown in Table 2 of Example 1.

The cosmetic compositions of urea and ammonium salt are useful themselves in cosmetic treatments and can also be used with additional ingredients to prepare other cosmetic formulations wherein aqueous urea is a useful ingredient.

For example, a cosmetic composition of this invention can be used alone as a put-on leave-on treatment for hair.  The pH value of a put-on leave-on product is typically about 6 to about 7. Such treatments can increase the bulk of the hair while also providing improved static control and moisturization.  An illustration of such a use is shown in Example 4.

Another put-on leave-on hair treatment of this invention includes the cosmetic composition and also typically contains a conditioning agent for hair as is known in the art.  Illustrative, useful conditioning agents include, but are not limited to quaternary nitrogen-containing compounds such as stearyldimethylbenzylammonium chloride, di-iso-cetyldimethylammonium chloride, polymers that contain quaternary nitrogen groups such as polydiallyldimethylammonium chloride, silicones such as the high molecular weight dimethylpolysiloxanes and the so-called "volatile silicones" such as the dimethyl cyclosiloxanes that contain 3-6 dimethylsiloxy groups and the like.  Additional ingredients usually found in conditioners such as perfume, colorant, thickeners, preservatives and the like can likewise be included.

For put-on leave-on hair treatments, it is particularly preferred to utilize urea at about 10 to about 30 weight percent of the final composition, or at about 1.5 to about 5 molar.

-10-

Another exemplary use of a cosmetic composition of this invention is as a skin moisturizer having a pH value of about 6 to about 7. Here, it is thought that the keratin swelling and humectant properties of urea may play a role in providing a beneficial result. A skin moisturizer of this invention includes the cosmetic composition and can additionally contain an emollient for skin such as mineral oil, petrolatum, jojoba oil and the like, as well as emulsifying agents, perfumes, thickener and the like are normally included with the product.

A particular utility for the cosmetic compositions of this invention is as a component in the lotion of reductive hair curling (waving) and straightening systems. Since the chemistry involved in hair curling and straightening is substantially similar, both processes will be referred to hereinafter as "waving".

The word "reductive" is meant herein to differentiate the present method of waving from those hair treating processes that are carried out at high pH values, e.g. at about pH 11 to about pH 14. The high pH treatments occur primarily by the transformation of disulfide bonds in hair keratin cystine groups into lanthionine groups, while the reductive treatments are believed to occur by breaking and then reforming the hair keratin disulfide bonds.

When used in waving preparations, the cosmetic compositions of this invention comprise a separately packaged component of a hair waving system that is admixed with at least a second component of the hair waving system to form the waving lotion. The second separately packaged component of the hair waving system includes a reductive hair disulfide bond-breaking agent and is called a base lotion.

-11-

For use in reductive waving compositions, the urea is most preferably present in its package in an amount of about 45 to about 60 weight percent, or about 8 to about 11 molar. However, the concentration of urea in the activator can be such as to provide the waving lotion produced by admixture of the activator and base lotion with about 4 to about 25 weight percent urea. In addition, an amount of ammonium salt sufficient to adjust the urea solution to a pH value of about 6.5 to about 7.5 is more preferably utilized.

Reductive hair waving is broadly old in the art so the details of the process steps and chemistry believed to be involved will not be dealt with herein. In addition, the reactants, their concentrations, times and temperatures known in the art are broadly useful herein and will be dealt with broadly.

The particularly preferred reducing agents for use in a second, separately packaged component of the waving lotion system or hair waving product are water-soluble thiol-containing agents such as mercaptocarboxylic acids, their alkali metal and ammonium salts, their esters and particularly glyceryl esters, and mixtures thereof. Examples of these reducing agents include thioglycolic acid, thiolactic acid, 3-mercaptopropionic acid and the like. Suitable alkali metal salts of these acids include sodium or potassium salts. Ammonium salts can also be used, and include salts prepared from these acids and ammonia, mono- di- or triethanolamine and mono-, di- or tri-iso-propanolamine. Particularly preferred mercaptocarboxylic acid esters include glyceryl thioglycolate.

Bisulfite ion, present in the waving lotion as a water-soluble alkali metal or ammonium

-12-

bisulfite, is also a particularly preferred reducing agent herein. The cleavage of hair disulfide bonds using the bisulfite ion in waving processes is sometimes referred to as "sulfitolysis." Since bisulfite ion is a well known reducing agent, and since its action upon the hair includes production of hair keratin thiol groups which remain in the hair during waving, bisulfite ion is included herein as a reducing agent.

The reducing agent capable of breaking disulfide bonds is present in aqueous solution in the separately packaged component of this waving lotion system in an amount that is sufficient to wave the hair after admixture and dilution with the cosmetic composition component of the system. In usual practices, the reducing agent is present at from 0.5 to about 30 percent by weight of the admixed waving lotion, as used on the hair.

When the particularly preferred thiol-containing acids or acid salts are utilized, they are preferably present at about 1 to about 20 weight percent of the waving lotion, measured as the free acid. Thiol-containing esters, such as glyceryl thioglycolate, are particularly preferred to be utilized at about 3 to about 30 weight percent of the waving lotion. Alkali metal or ammonium bisulfites are preferably present in the waving lotion at from about 5 to about 15 weight percent, and more preferably from about 10 to about 13 weight percent.

The pH values of waving lotions, after admixture with the cosmetic composition, useful herein, are dependent upon the reductive waving agent utilized, and are broadly about 6.5 to about 9.5. For example, compositions, which include mercaptocarboxylic acid salts, such as ammonium

-13-

thioglycolate, typically have a pH value of from about 6.5 to about 9.5, while compositions including mercaptocarboxylic acid esters, such as glyceryl thioglycolates typically have pH values of about 6.5 to about 7.5. Waving lotions that contain bisulfite ion, such as those containing ammonium bisulfite, are typically used at a pH value of about 6.5 to about 9.

In a usual waving process, the hair is typically first shampooed and towel blotted. The waving lotion is then applied and maintained in contact with the hair for period of time sufficient to form partially reduced hair; i.e., to break enough of the hair keratin disulfide bonds to produce a change in configuration if that hair is wound on rollers and then neutralized. The amount of time required to produce a permanent wave using the above described compositions and agents is well known in the art.

After the partially reduced hair is formed, the waving lotion is rinsed substantially completely from the partially reduced hair. The amount of rinsing needed at this step is that amount known to skilled practitioners in the art, and is the amount used when the waving lotion is rinsed from the hair in a conventional waving process prior to the neutralization step. In usual practice, about three minutes of rinsing in a sink using running warm tap water is sufficient to rinse the hair substantially free of the first, reducing agent-containing, composition. As used herein, the term "rinse" in any of its grammatical forms includes treatment with a shampoo.

The hair so treated is usually strained by wrapping it upon rollers of a diameter chosen to impart a tight or loose amount of curl. Wrapping of the hair on the rollers can be accomplished at any time prior to the neutralization step (discussed

-14-

below) in which the disulfide bonds are reformed. The treated hair can also be waved without the use of rollers so that the weight of the wet hair provides straightening strain in what is called "straight waving." Wrapping on rollers or straight waving changes the configuration of the partially reduced hair.

The final step required in waving is neutralization of the partially reduced hair by the application of a neutralizing composition to rebuild the broken hair keratin disulfide bonds to form the waved hair. Several means of rebuilding hair keratin disulfide bonds which have been cleaved by reducing agents are known in the art. For example, when thiol-containing agents (acids, acid salts or esters) are utilized to break the disulfide bonds, oxidizing agents, such as hydrogen peroxide, perborate or bromate salts, such as sodium perborate and sodium bromate, are well known to be capable of rebuilding broken hair keratin disulfide bonds.

When bisulfite ion is utilized as a reductive hair disulfide bond breaking agent, it is well known that the hair can be neutralized and the hair keratin disulfide bonds remade by treating the partially reduced hair with an aqueous composition having a pH value of from about 8.5 to about 10.5. Other agents such as water soluble disulfide salts like diammonium dithiodiglycolate can also be utilized in the reformation of hair disulfide bonds from partially reduced hair prepared using bisulfite ion, as can oxidizing agents, such as hydrogen peroxide.

The concentration of agents within the neutralizing composition and the pH value of that composition are well known to those skilled in the hair waving arts and need not be discussed further herein.

-15-

The cosmetic compositions of this invention are utilized in a manner similar to the way that a conventional cosmetic treatment of the same type is used. For example, as a put-on leave-on treatment for hair, the cosmetic composition is applied to the hair, spread through out the hair to contact the fibers, as by combing or massage, and maintained in contact with the hair fibers until the fibers are dry.

As a skin moisturizer, the cosmetic composition is applied to the skin and then spread over the area of skin to be moisturized to contact the skin, and maintained in contact with the skin until it has been absorbed. Of course, if too much moisturizer has been applied to be absorbed in a convenient period of time, the excess can be removed as by wiping with a towel or tissue.

When utilized as part of a hair waving system, the cosmetic composition is included in one of at least two separately packaged components. A second of the separately packaged components includes an aqueous solution of a reductive, hair disulfide bond-breaking agent. The separately packaged components are admixed to form the waving lotion which is then applied to the hair. The waving lotion so produced contains urea, an ammonium salt of an unreactive acid and the reductive disulfide bond-breaking agent in the amounts and at the pH values described hereinbefore.

After admixture of the separately packaged components of the hair waving system to form the waving lotion, that lotion is applied to the hair to contact the hair fibers and form partially reduced hair that contains broken disulfide bonds in the manner known in the art. Upon formation of the partially reduced hair, the hair is strained and then

-16-

rinsed to wash the waving lotion from the hair. The hair is thereafter neutralized by a method also well known in the art to reform the disulfide bonds within the hair.

Additional, separately packaged components can also be included with the hair waving system. The additional component may also be included within either package of the hair waving system.

An example of such an additional component is a conditioner, such as a solution of a polymeric material formed by the reaction of dimethyl sulfate and a copolymer of vinylpyrrolidone and dimethylaminoethyl methacrylate sold under the trademarks GAFQUAT 734 and GAFQUAT 755 by GAF Corporation. A more preferred polymeric conditioner is the polymer of hydroxyethyl cellulose reacted with epichlorohydrin and then quaternerized with trimethylamine that is sold under the trademarks JR-30M and JR-125 by Union Carbide Corporation.

The present invention also encompasses a method for retardation of the production of titratable alkali from an aqueous solution containing urea. Since hydrolysis of urea in water produces ammonia and an increase in titratable alkali in the solution, retardation of the production of titratable alkali is thought to also illustrate the retardation of urea hydrolysis, and thus is a measure of the improved stability of the urea.

The method of retarding the production of titratable alkali from an aqueous solution of urea comprises combining in aqueous solution a sufficient quantity of an ammonium salt of an unreactive acid with urea to adjust the pH value of the combined solution so formed to about 6 to about 8, maintaining the pH value of that combined solution at about 6 to about 8. The pH value is more preferably adjusted to and maintained at a pH value of about 6.5 to about

-17-

7.5. The amounts of urea, or substituted urea, and ammonium salt utilized in this method are the amounts of both ingredients discussed before in regard to the cosmetic composition and its ingredients. It is noted that the pH value can be additionally adjusted to the desired range by use of small amounts of an externally supplied acid and/or base.

This invention is further illustrated by the Examples that follow.

Best Mode For Carrying Out the Invention


Retardation of Production of Titratable
Example 1:    Alkali in Solutions That Contain Urea

The retardation of the production of titratable alkali and presumed urea hydrolysis was determined for aqueous solutions that contained 50 weight percent urea, alone or with additives. The number of milliequivalents (meq.) of free alkali per milliliter (ml) of solution was determined after storage by titration of a 10 ml urea solution with 1 normal (N) sulfuric acid to the methyl red endpoint (pH 5.8-6.0) using the following equation:

$$\text{Meq. of free alkali/ml of urea solution} = \frac{\text{ml of 1N } H_2SO_4}{10 \text{ ml}}$$

The urea solutions, without additives, had pH values ranging from 8.8 to 9.8 when prepared in deionized water. Addition of the additives adjusted the pH values of those urea solutions to values of 6.5 to 7.9. The solutions for which the free alkali determinations were made were aged at a temperature of 43°C. (110°F.) for up to 8.5 months or at a temperature of 60°C. (140°F.) for up to 9 weeks. The results of

-18-

the determinations are given in Tables 1 and 2, below, for aging at 43°C. and 60°C, respectively.

Table 1
Production of Alkali From Urea
Solutions Aged at 43°C.

| Additive In Urea Solution[1] | Initial pH Value | Aging Time (Months) | Meq. Alkali/ml Urea Solution | |
|---|---|---|---|---|
| | | | Initial | After Aging |
| None | 9.8 | 1.5 | 0.005 | 0.07 |
| None | 9.3 | 3.5 | 0.005 | 0.28 |
| None | 9.3 | 7 | 0.005 | 0.50 |
| None | 8.8 | 8.5 | 0.003 | 0.62 |
| Ammonium acetate (0.16M) | 7.8 | 1.5 | 0.018 | 0.03 |
| Ammonium chloride (0.5M) | 7 | 5 | 0.005 | 0.02 |
| HEEDTA[2] (0.1 wt.%) | 7.2 | 4.5 | 0.005 | 0.24 |
| HEEDTA[2] + Ammonium chloride (0.1 wt.% + 0.19M, respectively) | 7.2 | 4.5 | 0.005 | 0.04 |
| Citric acid (0.01 wt.%) | 6.5 | 8.5 | 0.000 | 0.60 |
| Citric acid (0.005 wt.%) | 7.5 | 8.5 | 0.000 | 0.61 |

Note 1: All solutions contained 50 weight percent urea in deionized water.

Note 2: HEEDTA is N-hydroxyethylethlene-diaminetriacetic acid sold under the trademark HAMP-OL ACID by W.R. Grace. Addition of HEEDTA in the amounts shown brought the pH value of both solutions in which it was used to about 5.6. The pH value of the solutions was adjusted to 7.2 by the addition of ammonium hydroxide.

-19-

## Table 2
### Production of Alkali From Urea Solutions Aged at 60°C.

| Additive in Urea Solution[1] | Initial pH Value | Meq. Alkali/ml Urea Solution at Various Times | | |
|---|---|---|---|---|
| | | Initial | 3 Weeks | 9 Weeks |
| None | 9.5 | 0.005 | 0.40 | 1.13 |
| Ammonium Chloride (0.14M) | 7.9 | 0.005 | 0.17 | 0.66 |
| Ammonium Chloride (0.22M) | 7.1 | 0.005 | 0.10 | 0.38 |
| Ammonium Sulfate (0.23M ammonium ion) | 7.3 | 0.005 | 0.06 | 0.15 |
| Sodium chloride (0.26M) | 9.0 | 0.005 | 0.46 | 1.17 |
| Citric acid (0.005 wt.%) | 7.5 | 0.005 | 0.47 | 1.23 |
| Citric acid (0.01 wt.%) | 6.5 | 0.000 | 0.43 | 1.23 |

Note 1: All solutions contained 50 weight percent urea in deionized water.

The data in the above Tables illustrate that the ammonium chloride, ammonium sulfate and ammonium acetate retard the production of titratable alkali from the urea solutions. The data also show that the reduction of titratable alkali is not merely a result of the lowered pH value caused by the ammonium salt in that addition of citric acid to adjust the solution pH value to the range obtained by addition of ammonium salt did not retard production of alkali, but actually increased that production.

-20-

HEEDTA, a sequestering agent, was added in small amounts, 0.1 weight percent, to a urea solution to retard urea decomposition that might be caused by metal ion catalysis. The results using HEEDTA alone were equivocal as to protection from metal ion catalysis.

The production of titratable alkali was substantially reduced when the same amount of HEEDTA and 0.19 molar ammonium chloride, a particularly preferred ammonium salt, were admixed together with the same amount of urea and at the same pH value as had been used in the determination utilizing HEEDTA alone.

The fact that solutions containing sodium chloride at about the same molar concentration as the ammonium salts produced slightly more titratable alkali at a slightly lower pH value than the urea solution alone is believed to indicate that the ammonium salts useful herein do not reduce the production of titratable alkali by increasing the ionic strength of the urea solutions.

Example 2: Waving Lotion System

A waving lotion system was prepared containing a separately packaged cosmetic composition of this invention and a separately packaged composition including an agent for reductively breaking disulfide bonds in hair.

The cosmetic composition was prepared as follows:

| | Ingredient | Weight Percent |
|---|---|---|
| 1. | Deionized water | 48.8 |
| 2. | Urea (prilled) | 50.0 |
| 3. | Ammonium chloride | 1.2 |
| | | 100.0 |

-21-

The water was heated to a temperature of 60° C. (about 140° F.) and the urea was added with agitation to provide a solution. The resulting solution was reheated to a temperature of 40° C. (about 105° F.) and the ammonium chloride was admixed. The resulting admixture was agitated until a substantially homogeneous, clear, colorless solution resulted which had a pH value of about 6.9-7.3 over several preparations.

The aqeuous solution containing the reductive disulfide bond-breaking agent was prepared as follows:

| Ingredient | Weight Percent |
|---|---|
| 1. Deionized water | 71.53 |
| 2. Ammonium thiglycolate (60 perent active as thioglycolic acid) | 26.67 |
| 3. Polyoxyethylene (15) nonyl phenyl ether | 0.88 |
| 4. Perfume | 0.22 |
| 5. Ammonium hydroxide (28%) to about pH 7.1 | 0.70 |
| | 100.00 |

Ingredients 1 and 2 were admixed until substantially homogeneous. A premixture of ingredients 3 and 4 was prepared and then added to the admixture formed from ingredients 1 and 2 with stirring to form a substantially homogeneous resulting admixture. Ingredient 5 was thereafter added slowly with agitation and the pH value of the composition was adjusted to 6.9-7.3 to provide a

-22-

clear, colorless solution of disulfide bond-breaking agent.

Each of the compositions so prepared was packaged separately in plastic containers to provide a waving lotion system. The package of the cosmetic composition, or activator contained 27.5 grams of that composition. The second bottle which contained the reductive, disulfide bond-breaking agent, ammonium thioglycolate, and termed a base lotion contained 82.5 grams of its composition.

Admixture of the contents of both of the above bottles provided a clear, colorless waving lotion solution of this invention that contained about 11.8 to about 12.2 weight percent ammonium thioglycolate, calculated as thioglycolic acid, about 12 to about 12.5 weight percent urea and about 0.29 to about 0.3 weight percent ammonium chloride. The pH value of the waving lotion averaged about 6.9 to about 7.3 over several preparations.

Hair waved with this lotion using conventional curling rollers, and heat provided by a salon-type hairdryer, followed by rinsing and neutralization produced curls that were tight and springy both on the day of waving and one week later after a shampoo treatment. Models having normal as well as tinted hair were used in this waving determination.

Storage of another package of the same cosmetic composition at 110°F. (about 43°C. for 4.5 months) showed a change in titratable alkali from an initial value of about 0.005 milliequivalents per milliliter to about 0.02 milliequivalents per milliliter. Thus, the stability imparted by the ammonium chloride was again demonstrated.

-23-

Use of the waving system and waving lotion prepared in this Example on dozens of models' hair provided excellent waving results on both tinted and normal hair.

Put-On Leave-On
Example 3: Hair Treatment

A cosmetic composition of this invention was used as put-on leave-on treatments for improving the weight, feel, static control and moisture content of hair.

Tresses of human hair used for these determinations were of two hair types: (1) normal, (unchemically treated) brown hair and (2) bleached hair. (Hair samples were obtained from DeMeo Brothers, New York).

Each type of hair was equilibrated at ambient temperatures under relative humidity (RH) conditions of 60% and then weighed. An aqueous cosmetic composition (A) of this invention containing 20 weight percent urea and 1 weight percent ammonium chloride (about 0.19 molar) was applied to the hair and worked through the hair. The treated hair was then towel blotted, dried, again equilibrated at 60% RH and re-weighed to obtain the amount of weight add-on, the treated hair was then placed at ambient temperature under relative humidity conditions of 90% RH for forty hours and again weighed to obtain percent moisture gain.

The weight add-on and moisture gain measured is shown in Table 3 compared to control hair treated only with water.

-24-

## Table 3
### Weight Add-on and Moisture Gain
### from a Put-on Leave-on Treatment

| Hair Type | Treating Composition | Percent Weight Add-on | Percent Moisture Gain |
|-----------|---------------------|----------------------|----------------------|
| Normal | A | +9.0 | 22.7 |
| Bleached | A | +9.6 | 25.9 |
| Normal | Water | -0.15 | 9.2 |
| Bleached | Water | -0.20 | 8.3 |

Besides evaluating the add-on and moisture gain, the treated hair was evaluated subjectively for body, bulk, and for static fly-away. The hair treated with Composition A was judged as having more body, more bulk, and less static flyaway than the control hair treated only with water.

### Example 4: Skin Moisturizing Composition

A put-on leave-on skin moisturizer, of this invention was prepared that contained 20 weight percent urea, 0.5 weight percent ammonium chloride (about 0.09 molar), 0.5 weight percent METHOCEL J5MS (a hydroxypropyl methylcellulose sold by The Dow Chemical Company) with the remaining weight being constituted by deionized water.

The subject's hands were washed with a conventional hand soap and then dried. The above composition was applied to one hand and rubbed over and into the skin, while the other hand was treated with water. One-half hour after the application of the moisturizer, both hands were examined by two expert evaluators. The hand treated with the put-on leave-one product of this invention was adjudged to look more moisturized and felt softer to the touch than did the water-treated hand.

-25-

The determination was thereafter repeated, switching the hand that received the moisturizer treatment and the hand treated with water. Again, both evaluators chose the correct hand as having had the moisturizing treatment.

The present invention has been described with respect to preferred embodiments. It will be clear to those skilled in the art that modifications and/or variations of the disclosed compositions and methods can be made without departing from the scope of the invention set forth herein. The invention is defined by the claims that follow.

## C L A I M S

1. A cosmetic composition comprising an aqueous solution of a urea compound that is selected from the group consisting of urea, lower alkyl-substituted urea, and mixtures thereof present at a concentration of about 0.5 to about 12 molar, and an amount of an ammonium salt of an unreactive acid sufficient to adjust the pH value of the urea compound solution to about 6 to about 8, said cosmetic composition being stable to the production of titratable alkali.

2. A put-on leave-on treatment for hair including the cosmetic composition of claim 1 and additionally containing a hair conditioning agent.

3. A skin moisturizer including the cosmetic composition of claim 1, and additionally containing an emollient for skin.

4. A hair waving system comprising two separately packaged components, the first of the packaged components including the cosmetic composition of claim 1 and the second of the packaged components including an aqueous solution of a hair disulfide bond-breaking agent, said separately packaged components when admixed providing a waving lotion that contains about 4 to about 25 percent by weight urea compound, a sufficient amount of the reductive disulfide bond-breaking agent to wave hair, and having a pH value of about 6.5 to about 9.5.

5. A method of retarding the production of titratable alkali from an aqueous solution containing a urea compound that is selected from the group consisting of urea, lower alkyl-substituted urea and mixtures thereof comprising combining in said solution a sufficient quantity of an ammonium salt of an unreactive acid to adjust the pH value of the

-2 -

solution to about 6 to about 8 and to maintain said adjusted pH value.

6. A method of retarding the production of titratable alkali from an aqueous solution containing about 0.5 to about 12 molar urea comprising combining in said solution an ammonium salt of an unreactive acid in an amount of about 0.02 to about 1 molar to form a combined solution having a pH value of about 6 to about 8, and maintaining said combined solution at a pH value of about 6 to about 8.

7. A method of treating hair to provide increased weight, bulk, static control and moisturization to said hair comprising the steps of applying the composition of claim 1 to said hair to contact the hair fibers and maintaining said contact until said hair is dry.

8. A method of treating hair to provide conditioning to said hair comprising the steps of applying the composition of claim 2 to said hair to contact the hair fibers and maintaining said contact until said hair is dry.

9. A method of moisturizing skin comprising the steps of applying the composition of claim 3 to said skin, spreading said composition over the area of the skin to be moisturized to contact that skin with said composition, and maintaining said contact until said moisturizer is absorbed by the skin.

10. A method of waving hair comprising the steps of:

providing the hair waving system of claim 4;

admixing the two separately packaged components of said hair waving system to form a waving lotion;

applying said waving lotion to hair to form partially reduced hair that contains broken disulfide bonds;

straining said partially reduced hair; and
applying a neutralizer to the strained,
partially reduced hair to reform the disulfide bonds.

1/7

PH VALUES OF AQUEOUS UREA
SOLUTIONS AS A FUNCTION
OF REAGENT CONCENTRATION

△ = AMMONIUM CHLORIDE (50% UREA)

⬡ = AMMONIUM CHLORIDE (20% UREA)

□ = AMMONIUM ACETATE (50% UREA)

● = AMMONIUM SULFATE (50% UREA)

◇ = CITRIC ACID (50% UREA)

▼ = SODIUM CHLORIDE (50% UREA)

PH OF UREA SOLUTION

MOLARITY OF REAGENT CATION OR CITRIC ACID